(19) Europäisches Patentamt
European Patent Office
Office européen des brevets

(11) Publication number: 0 196 001
B1

(12) EUROPEAN PATENT SPECIFICATION

(45) Date of publication of the patent specification: 25.01.89

(51) Int. Cl.⁴: C 07 D 333/22, C 07 D 333/28, A 61 K 31/38

(21) Application number: 86103651.5

(22) Date of filling: 18.03.86

(54) **Thiophene ring-substituted alpha-(alkylaminopropionyl)-thiophene derivatives, a process for preparing them and pharmaceutical compositions containing them.**

(30) Priority: 20.03.85 ES 541911
20.02.86 ES 552220

(43) Date of publication of application:
01.10.86 Bulletin 86/40

(45) Publication of the grant of the patent:
25.01.89 Bulletin 89/4

(84) Designated Contracting States:
BE CH DE FR GB IT LI NL

(56) References cited:
FR-A-2 453 172
GB-A-1 044 964
GB-A-1 313 150

CHEMICAL ABSTRACTS, vol. 80, no. 9, 4th March 1974, page 348, no. 47831p, Columbus, Ohio, US; & ES - A - 381 478 (LABORATORIOS MADE S.A. et al.) 16-11-1972
CHEMICAL ABSTRACTS, vol. 87, no. 13, 26th September 1977, page 604, no. 102152q, Columbus, Ohio, US; & ES - A - 411 086 (LABORATORIOS MADE S.A.) 16-07-1976

(73) Proprietor: FERRER INTERNACIONAL, S.A., Gran Via Carlos III, 94, 08028 Barcelona (ES)

(72) Inventor: Foguet, Rafael, Llusanés 8, ES- 08022 Barcelona (ES)
Inventor: Forné, Ernesto, Dr., Felipe de Paz 15, ES-08028 Barcelona (ES)
Inventor: Sacristán, Aurelio, Santa Amelia 2, ES-08034 Barcelona (ES)
Inventor: Ortiz, José A., Dr., Córcega 429, ES-08037 Barcelona (ES)

(74) Representative: Kinzebach, Werner, Dr., Patentanwälte Reitstötter, Kinzebach und Partner Sternwartstrasse 4 Postfach 86 06 49, D-8000 München 86 (DE)

EP 0 196 001 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

LIBER, STOCKHOLM 1989

## Description

The present invention relates to thiophene ring-substituted α-(alkylaminopropionyl)-thiophene derivatives of the general formula (I):

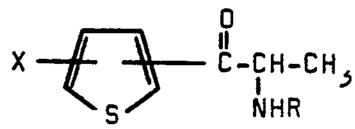

(I)

wherein X is a group different from hydrogen selected between methyl or chlorine and R is a linear or branched chain alkyl group having 3 to 4 carbon atoms, and the nontoxic addition salts thereof, as well as to a process for preparing these compounds and to pharmaceutical compositions containing these compounds.

The preferred addition salts are the hydrochlorides.

The compounds of the present invention may be prepared according to the following scheme:

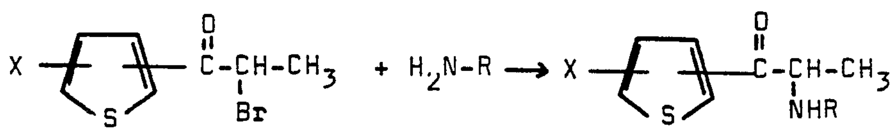

(II) (III) (I)

Bromine displacament in the compounds of formula II, where in X is as defined above for (I), by the amines of formula (III), wherein R is as defined above for (I), occurs in an inert medium, preferably acetonitrile, and at a maximal temperature of 50°C, followed by vacuum evaporation of solvent, removal and evaporation to dryness of extracts. From the residue thus obtained, corresponding to the compounds of formula (I), it is possible to form their pharmaceutically acceptable salts, preferably hydrochlorides, by treating the respective acids in an inert solvent; aliphatic-chlorinated hydrocarbons especially methylene chloride, or ethers, especially diethyl ether, are preferred.

Then the formed precipitate is separated by filtration. Neutralization of respective salts allows to obtain the compounds of this invention as free bases.

α-(Bromopropionyl)-thiophene precursors of general formula (II) are obtained by conventional methods in Organic Chemistry; the reaction of propionyl-thiophenes with bromine in a medium composed by an aliphatic-chlorinated hydrocarbon, for example, methylene chloride, chloroform or carbon tetrachloride, are preferred. In turn, propionyl-thiophenes are susceptible to be obtained either by acylation of respective thiophenes with a derivative of propionic acid, preferably propionyl chloride, under suitable conditions of Friedel-Crafts' reaction, or by reacting lithium derivatives of respective thiophenes with propionitrile. When X is chlorine, starting compounds such as commercially-available chlorothiophenes or dichlorothiophenes are, if possible, used, and the substituent X = Cl may be introduced into the synthesis sequence on intermediate steps depending on the desired position. Sulphuryl chloride is preferred as chlorinating agent. When X is methyl, respective methylthiophenes are, if possible, also used. The preparation of α-(bromo propionyl)-thiophene precursors pf formula (II) is hereinafter illustrated.

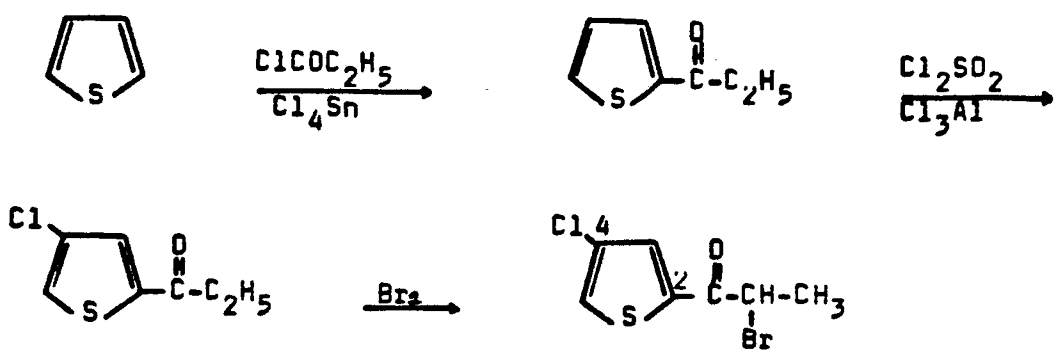


(II, X= 4-Cl; chain position= 2)

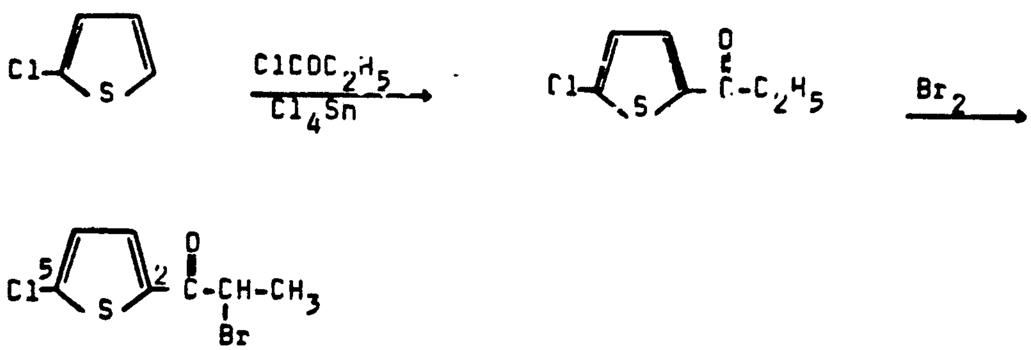


(II, X= 5-Cl; chain position= 2)

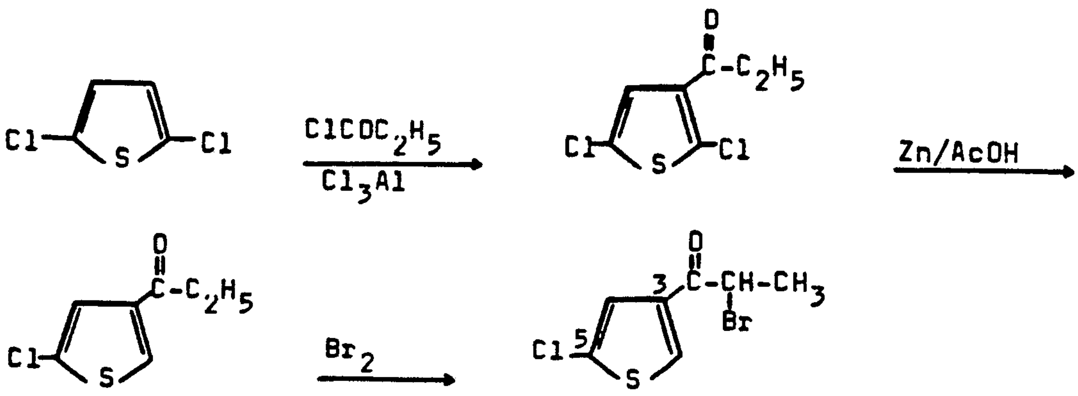


(II, X= 5-Cl; chain position= 3)

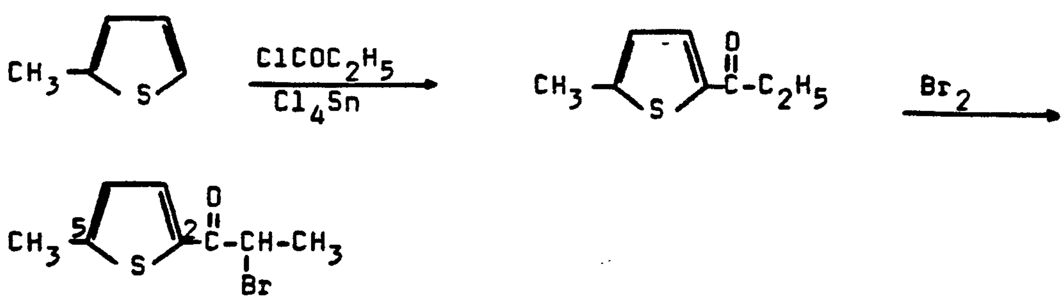


(II', X'= 5-$CH_3$, chain position= 2)

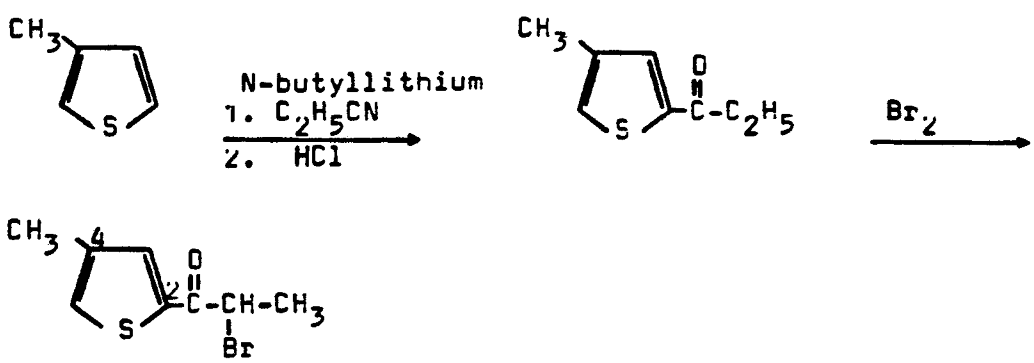


(II', X'= 4-$CH_3$, chain position= 2)

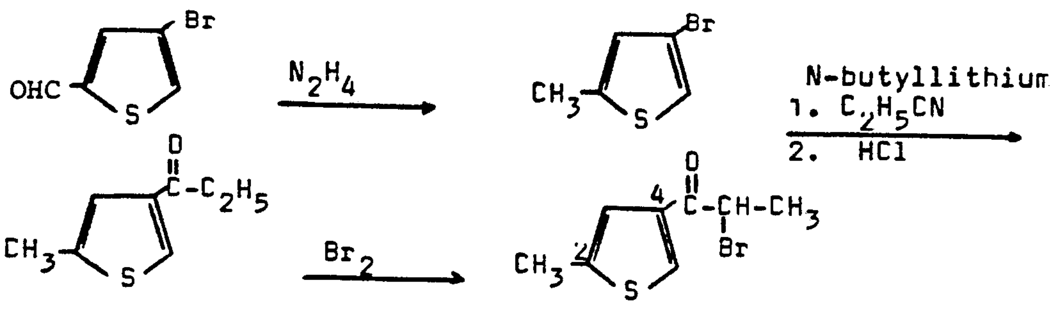


(II', X'= 2-CH3, chain position= 4)

The compounds of the present invention have an effective antidepressant activity as evidenced by R.D. Porsolt (Immobility behavioural despair test in mice: Arch.Int.pharmacodyn., 229, 327-336, 1977), which differ from the thiophene ring non-substituted α-(alkylaminopropionyl)-thiophenes, as disclosed in French Patent No. 3414M and British Patent No. 1 313 150, because these known compounds have anorexic activity devoided of central excitatory action or cardiovascular action, and neuroleptic, tranquillizing and analgesic action respectively. Consequently, the presence of an X group different from hydrogen, selected between methyl or chlorine, in the compounds of formula (I) leads to compounds having a very effective antidepressant action. Thus, the compounds of this invention are useful as a medicament for treating depressions.

The compounds of the present invention mixed with pharmaceutically acceptable carriers can be administered by the oral route in the form of tablets, capsules, coated tablets, syrups, solutions, etc., by injectable route and by rectal route at daily doses ranging from 2.5 to 250 mg/kg.

By way of a non-limitative illustration within the essence of the invention, some examples are described hereinbelow referring to the possible way to obtain (I) by following the steps of the disclosed process.

**Example 1**: 2-Propionylthiophene

400 g of thiophene and 437.33 g of propionyl chloride in 2850 ml of anhydrous benzene are introduced into a 6-liter flask fitted up with a mechanical stirrer. Then the mixture is cooled at 0-5°C in ice-salt bath and by keeping this temperature, 1343.7 g of tin tetrachloride in 570 ml of anhydrous benzene are added (approximate addition time: 2 hours). Cooling bath is removed and the mixture is stirred for 20 hours at room temperature, then it is poured onto 3.8 kg of ice and 1 liter of water and vigorously stirred for 1 hour. The aqueous phase is decanted and extracted with 1.5 liters of ethyl ether; the organic extracts are washed with water to neutralization and filtered off. After evaporation of solvent and vacumm distillation of the residue, 560 g (yield: 85 %) ofa colourless liquid are obtained. Boiling point: 77-79°/0.7-0.8 torr, $n^{20}_D$ = 1.5530, GLC: 100 % and analysis correct.

IR Spectrum (film), $cm^{-1}$: 3090, 3000-2880, 1660, 1410, 1220, 1050, 900, 845, 715.

$^1$H-NMR ($CCl_4$), ppm: 1.16 (t, 3H, J=7 Hz; $CH_3$-), 2.83 (q, 2H, J=7 Hz; -$CH_2$-), 7.03 (dd, 1H, $J_{3,4}$=3.75 Hz, $J_{4,5}$=4.75 Hz; $H_{Tiophene}$), 7.50 (dd, 1H, $J_{4,5}$=4.75 Hz, $J_{3,5}$=1 Hz, $H_{Thiophene}$) and 7.59 (dd, 1H, $J_{3,4}$=3.75 Hz, $J_{3,5}$=1 Hz, $H_{Thiophene}$).

**Example 2**: 4-Chloro-2-propionylthiophene

To a suspension of 400.02 g of aluminium chloride in 200 ml of dry chloroform under cooling at 10-20°C, 280.4 g of 2-propionyl-thiophene are added under vigorous stirring. The mixture is then brought to 25-30°C and 350.92 g of newly distilled sulfuryl chloride are slowly added (3.5 hours), then heated in water bath at 40-42°C for 1 hour and left to stand overnight at room temperature. Thereafter, it is slowly poured onto 2.8 kg of ice under stirring, the aqueous phase is extracted with 1.2 and 0.5 liters of methylene chloride and the organic extracts are jointly washed with water (three times), sodium bicarbonate saturated solution, and water again to neutralization. After evaporation of solvent, the residue (370.5 g) under rectifying column ($L_0$=19 cm) with Raschig rings gives 216.6 g and recrystallizes from hexane (350 ml) to yield 157.4 g (45 %) of a white solid with m.p. 58-60°C, GLC: 98 %.

IR Spectrum (KBr) $cm^{-1}$: 3080, 3000-2880, 1665, 1400, 1220, 1190, 910.

$^1$H-HMR Spectrum ($CCl_4$), ppm: 1.16 (t, 3H, J=7 Hz; $CH_3$-), 2.81 (q, 2H, J=7 Hz; -$CH_2$-), 7.29 (d, 1H, J=1.4 Hz; $H_{Thiophene}$) and 7.43 (d, 1H, J=1.4 Hz; $H_{Thiophene}$).

**Example 3**: α-Bromo-4-chloro-2-propionylthiophene

To a solution of 60 g of 4-chloro-2-propionylthiophene in 280 ml of dry methylene chloride under stirring and cooled at 0-5°C, the equimolecular amount of bromine (54.9 g) dissolved in 135 ml of dry methylene chloride is slowly added; as the reaction starts, decolorization of bromine occurs immediately while adding the reaction mixture. After completing the addition, the bath is removed, stirring for further 30 minutes is performed, the methylene chloride is evaporated under vacuum and the liquid residue is used without further purification.

IR Spectrum (film), $cm^{-1}$: 3100, 1665, 1400, 1230, 1155, 980, 910, 840.

$^1$H-NMR Spectrum ($CDCl_3$), ppm: 1.84 (d, 3H, J=6.6 Hz; $CH_3$-), 4.93 (q, 1H, J=6.6 Hz, -CH < ), 7.36 (d, 1H, J=1.25 Hz, $H_{Thiophene}$) and 7.59 (d, 1H, J=1.25 Hz; $H_{Thiophene}$).

**Exemple 4**: 2-[α-(tert-butylaminopropionyl)]-4-chlorothiophene hydrochloride

To 87.69 g of α-bromo-4-chloro-2-propionylthiophene in 100 ml of acetonitrile, 60.7 g of tert-butylamine in 100 ml of acetonitrile are slowly added without exceeding 32°C (water bath regulation). As the exothermia diminishes, the mixture is left under stirring for 20 hours at room temperature. The solvent is evaporated at vacuum (below 35°C), taken in methylene chloride (300 ml) and water (300 ml); the aqueous phase is removed with methylene chloride, the organic extracts are washed four times with 200 ml of water, dried and evaporated to dryness. The residue (80.7 g) is dissolved in 600 ml of dry methylene chloride, cooled with ice-salt bath at 0°C and a dry HCl (g) stream is slowly spread. The precipitated hydrochloride is filtered off, washed twice with dry methylene chloride and dried at vacuum to give 52.7 g of white solid (m.p. 245-250°C) which, after recrystallizing from ethanol, yields 28 g (30 %) of the hydrochloride (m.p. 250-255°C); analysis correct.

IR Spectrum (KBr), $cm^{-1}$: 3420, 3100-2400, 1675, 1545, 1400, 1240, 1205, 1125, 895, 795.

$^1$H-NMR Spectrum ($CD_3DD$), ppm: 1.39 (s, 9H; -C$(CH_3)_3$, 1.67 (d, 3H, J=7 Hz; $CH_3$-), 5.14 (q, 1H, J=7 Hz; -CH < ), 7.97 (d, 1H, J=1.75 Hz; $H_{Thiophene}$).

Neutralization of the hydrochloride allows to isolate respective base, m.p. 79-81°C (hexane) and analysis correct.

**Example 5**: 5-Chloro-2-propionylthiophene

To a solution of 77.44 g of 2-chlorothiophene and 60.42 g of propionyl chloride in 390 ml of anhydrous benzene under cooling at 0-5°C (ice-salt bath), 184.56 g of tin tetrachloride in 78 ml of anhydrous benzene are dropwise added for about 1 hour and at the above temperature while separating a redish solid. Then it is stirred for 20 hours at room temperature, poured onto 520 g of ice and 150 g of water under stirring and 15 minutes later the phases are decanted: the aqueous phase is extracted with 250 ml of ethyl ether and the organic extracts are washed (3 times) with sodium chloride saturated solution. The crude product (105.3 g) is rectified by Vigreux Column ($L_0$= 11 cm) thus obtaining 75.80 g (yield: 65 %) of a white solid, m.p. 47-49°C, GLC: 100 % and analysis correct.

IR Spectrum (KBr), $cm^{-1}$: 3080, 3000-2860, 1650, 1410, 1210, 1010, 880, 780.

$^1$H-NMR Spectrum ($CCl_4$), ppm: 1.14 (t, 3H, J=7.6 Hz; $CH_3$-), 2.75 (q, 2H, J=7.6 Hz, -$CH_2$-), 6.85 (d, 1H, J=4 Hz, $H_{Thiophene}$) and 7.37 (d, 1H, J=4 Hz; $H_{Thiophene}$).

**Example 6**: α-Bromo-5-chloro-2-propionylthiophene

To a solution of 112.37 g of 5-chloro-2-propionylthiophene in 500 ml of dry methylene chloride under stirring and cooling at 0-5°C, 102.83 g of bromine in 250 ml of methylene chloride are added. Processing as described in Ex. 3, 167.8 g of a liquid residue corresponding to the α-bromo derivative are obtained which is pure enough to be used in the following synthesis step.

IR Spectrum (film), $cm^{-1}$: 3100, 3010-2880, 1660, 1420, 1240, 1010, 890, 800, 740.

$^1$H-NMR ($CCl_4$) Spectrum, ppm: 1.81 (d, 3H, J=6.6 Hz; $CH_3$-), 4.90 (q, 1H, J=6.6 Hz; -CH < ), 6.91 (d, 1H, J=4 Hz; $H_{Thiophene}$) and 7.55 (d, 1H, J=4 Hz, $H_{Thiophene}$).

**Example 7**: 2-[α-(tert-butylaminopropionyl)]-5-chlorothiophene hydrochloride

To 81.50 g of α-bromo-5-chloro-2-propionylthiophene in 100 ml of acetonitrile, 56.76 g of tert-butylamine in 100 ml of acetonitrile are slowly added without exceeding 32°C. As the exothermia diminishes, the mixture is

left under stirring for 20 hours at room temperature. Processing as described in Ex. 4, 75.7 g of a crude product are isolated and respective hydrochloride in dry methylene chloride (700 ml) with HCl (g) is formed. The insolubilized hydrochloride (65.4 g, m.p. 246-248°C) is recrystallized from ethanol thus obtaining 44.4 g (yield: 49 %) of a white solid, m.p. 255-257°C and analysis correct.

IR Spectrum (KBr), $cm^{-1}$: 3440, 3110-2500, 1660, 1550, 1410, 1245, 1010, 880.

$^1H$-NMR Spectrum ($CD_3OD$), ppm: 1.37 (s, 9H; $-C(CH_3)_3$), 1.66 (d, 3H, J=7 Hz; $-CH_3$), 5.14 (q, 1H, J=7 Hz; -CH < ), 7.24 (d, 1H, J=4.25 Hz; $H_{Thiophene}$) and 8.19 (d, 1H, J=4.25 Hz; $H_{Thiophene}$).

Neutralization of the hydrochloride allows to isolate respective base, m.p. 72-75°C (hexane) and analysis correct.

**Example 8**: 2,5-Dichloro-3-propionylthiophene

To a solution of 65.1 g of 2,5-dichlorothiophene and 77.79 g of propionyl chloride in 325 ml of nitroethane, 63.5 g of aluminium chloride are slowly added in nitrogen atmosphere for about 20 minutes, thus causing the temperature of the mixture rise up to 37°C. Then, the mixture is stirred for 4 hrs, poured on to 500 g of ice and removed twice with 250 ml of ethyl ether. The organic extracts are consecutively washed with 3N hydrochloric acid (150 ml), water (2x100 ml), 3N sodium hydroxide (3x600 ml) and saturated sodium chloride solution till neutralization. After drying and evaporating the solvent, the solid residue (68.9 g) is distilled and 58.00 g (yield: 65 %) of a white solid are obtained; b.p. 96-105°C/1.2 torr, m.p. 63-66°C and analysis correct.

IR Spectrum (KBr), $cm^{-1}$: 3120, 3080, 3000-2860, 1680, 1660, 1430, 1370, 1190, 1175, 1015, 800.

$^1H$-NMR Spectrum ($CCl_4$), ppm: 7.12 (t, 3H, J=7 Hz; $CH_3$-), 2.83 (q, 2H, J=7 Hz; $-CH_2-$) and 7.15 (s, 1H; $H_{Thiophene}$).

**Example 9**: 2-Chloro-4-propionylthiophene

To a suspension of 53.28 g of 2,5-dichloro-4-propionylthiophene in 57 ml of glacial acetic acid and 181 ml of water, 53.31 g of zinc dust (2 equivalents) are added in the course of 5 minutes and thus the temperature rapidly rises up to 72°C; then it is cooled in water bath till reaching 55°C within 10 minutes. 250 ml of ethyl ether are added, the insoluble solid is filtered off and the aqueous phase is removed with ether again; the extracts are washed with water, saturated NaCl solution and dried with potassium carbonate. The residue (43.48 g), which is obtained after evaporating the solvent, recrystallizes from n-pentane to yield 28.9 g (65 %) of a white solid, m.p. 50-53°C, GLC: 100 % and analysis correct.

IR Spectrum (KBr), $cm^{-1}$: 3100, 3000-2800, 1665, 1435, 1180, 1000, 780.

$^1H$-NMR Spectrum ($CCl_4$), ppm: 1.13 (t, 3H, J=7 Hz; $CH_3$-), 2.75 (q, 2H, J=7 Hz, $-CH_2-$), 7.28 (d, 1H, J=1.5 Hz, $H_{Thiophene}$), and 7.68 (d, 1H, J=1.5 Hz; $H_{Thiophene}$).

**Example 10**: α-Bromo-2-chloro-4-propionylthiophene

To a solution of 30.92 g of 2-chloro-4-propionylthiophene in 140 ml of dry methylene chloride under stirring and heating at 0-5°C, 28.29 g of bromine in 70 ml of methylene chloride are added. Processing as described in Ex. 3, 44.9 g of a coloured liquid are obtained with analysis correct.

IR Spectrum (film), $cm^{-1}$: 3100, 3000-2900, 1680, 1425, 1210, 1150, 1005, 845.

$^1H$-NMR Spectrum ($CCl_4$), ppm: 1.80 (t, 3H, J=7.0 Hz; $CH_3$-), 4.82 (q, 1H, J=7.0 Hz; > -CH-), 7.35 (d, 1H, J=1.5 Hz, $H_{Thiophene}$) and 7.87 (d, 1H, J=1.5 Hz; $H_{Thiophene}$).

**Example 11**: 4-[α-(tert-butylaminopropionyl)]-2-chlorothiophene

To 44.50 g of α-bromo-2-chloro-4-propionylthiophene in 55 ml of acetonitrile, 31 g of tert-butylamine in 55 ml of acetonitrile are slowly added without exceeding 32°C. As the exothermia diminishes, the mixture is left under stirring for 20 hours at room temperature. Processing as described in Ex. 4, 42.4 g of a dark liquid are isolated and respective hydrochloride in methylene chloride (400 ml) at 0°C with HCl (g) is formed. The insolubilized product (37.10 g) is recrystallized from ethanol to yield 38.5 g (40 %) of a white solid, m.p. 257-259°C (d) and analysis correct.

IR Spectrum (KBr), $cm^{-1}$: 3450, 3100-2400, 1675, 1545, 1230, 1200, 1175, 1000, 830.

$^1H$-NMR Spectrum ($CD_3OD$), ppm: 1.40 (s, 9H, $-C(CH_3)_3$), 1.62 (d, 3H, J=7.0 Hz; $CH_3$-), 5.10 (q, 1H, J=7 Hz; > CH-), 7.52 (d, 1H, J=1.5 Hz; $H_{Thiophene}$) and 8.72 (d, 1H, J=1.5 Hz; $H_{Thiophene}$).

**Example 12**: 1-Propionyl-5-methylthiophene

To a solution of 53.50 g of 2-methylthiophene and 50.43 g of propionyl chloride in 300 ml of anhydrous benzene under cooling at 0-5°C in an ice-salt bath, 141.97 g of tin tetrachloride in 60 ml of anhydrous benzene are dropwise added for about 1 hour at the above temperature. Then it is stirred for 22 hours at room temperature to give a dark red mixture which is poured onto 400 g of ice and 100 g of water under vigorous stirring; after 1 hour, the aqueous phase is removed with 200 ml of ethyl ether and the organic extracts are washed three times with sodium chloride saturated solution. The crude product (76 g), which solidifies by standing, is distilled at vacuum and 68.24 g (81 %) are obtained, b.p. 85-95°C/0.5-0.6 torr and m.p. 32-34°C.

IR Spectrum (KBr), $cm^{-1}$: 3100-2880, 1660, 1455, 1230, 1050, 890, 790,

$^1H$-NMR ($CDCl_3$), ppm: 1.18 (t, 3H, J=8 Hz; $CH_3$-), 2.50 (d, 3H, J=1 Hz; $CH_3$- thiophene), 2.83 (q, 2H, J=8 Hz; -$CH_2$-), 6,75 (dd, 1H, J=1 Hz and J'=3.75 Hz; H-thiophene) and 7.50 (d, 1H, J=3.75 Hz; H-thiophene).

**Examples 13**: α-Bromo-2-propionyl-5-methylthiophene

To a solution ot 34.12 g of 2-propionyl-5-methylthiophene in 180 ml of dry methylene chloride under stirring and cooling at 0-5°C, 35.35 g of bromine in 90 ml of methylene chloride are gently added. After completing the addition, the bath is removed, stirred for further 30 minutes, the methylene chloride is evaporated at vacuum and the residue (53.27 g, 100 %) is used for the following operation without further purification.

IR Spectrum (film), $cm^{-1}$: 3100-2900, 1665, 1450, 1245, 855.

$^1H$-NMR ($Cl_4C$), ppm: 1.81 (d, 3H, J=7 Hz; $CH_3$-), 2.52 (s, 3H; $CH_3$-thiophene) 4.95 (q, 1H, J=7 Hz; -CH < ), 6.75 (dd, 1H, J=1 Hz, J'=3.75 Hz; H-thiophene) and 7.55 (d, 1H, J=3.75 Hz; H-thiophene).

**Example 14**: 2-[α-(tert-butylaminopropionyl)]-5-methylthiophene hydrochloride

To 44.5 g of α-bromo-2-propionyl-5-methylthiophene in 60 ml of acetonitrile, 36.57 g of tert-butylamine are gently added without exceeding 30°C (water bath adjustment). The solvent is evaporated at vacuum (below 35°C), taken in 100 ml of methylene chloride and 100 ml of water, and the aqueous phase is removed again with 75 ml of the solvent; the organic extracts are washed with water, dried and evaporated. The crude material (42.0 g) is dissolved in 400 ml of dry ethyl ether, and by cooling in water-ice bath a dry HCl (g) stream is spread; the precipitated hydrochloride is filtered off, washed with ether and dried at vacuum to give 35 g of a whitish solid which, after recrystallizing from ethanol, yields 19.7 g (40 %) of a white solid corresponding to the hydrochloride (m.p. 262-263°C).

IR Spectrum (KBr), $cm^{-1}$: 3100-2400, 1655, 1550, 1445, 1250, 1205, 1055, 800, 825.

$^1H$-NMR ($d_6$-DMSO), ppm: 1.35 (s, 9H; -$C(CH_3)_3$), 1.64 (d, 3H, J=7 Hz; $CH_3$-), 2.58 (s, 3H; $CH_3$-thiophene), 5.13 (q, 1H, J=7 Hz; -CH < ), 7.05 (d, 1H, J=4 Hz; H-thiophene) and 8.28 (d, 1H, J=4 Hz; H-thiophene).

**Example 15**: 2-[α-(iso-propylaminopropionyl)]-5-methylthiophene hydrochloride

From 53.27 g of α-bromo-2-propionyl-5-methyl-thiophene in 65 ml of acetonitrile and 34.0 g of isopropylamine, and processing as described in Ex. 14, 41.3 of crude material are isolated and respective hydrochloride (11.2 g) is recrystallized from ethanol to give 6.5 g (15 %) of a white solid, m.p. 230-233°C and elemental analysis correct.

IR Spectrum (KBr), $cm^{-1}$: 3100-2400, 1645, 1550, 1445, 1250, 1065, 860, 830.

$^1H$-NMR ($d_6$-DMSO), ppm: 1.30 (d, 6H, J=7 Hz; $CH(CH_3)_2$) 1.54 (d, 3H, J=7 Hz; $CH_3$-), 2.55 (s, 3Hz $CH_3$-thiophene), 3.25 (m, 1H, J=7 Hz; $\underline{C}H(CH_3)_2$), 5.05 (q, 1H; J=7 Hz; CO-CH-), 7.05 (d, 1H, J=4 Hz; H-thiophene) and 8.20 (d, 1H, J=4 Hz; H-thiophene).

**Example 16**: 2-[α-(sec-butylaminopropionyl)]-5-methylthiophene hydrochloride

From 24.16 g of α-bromo-2-propionyl-5-methylthiophene in 30 ml of acetonitrile and 20 g of sec-butylamine, and processing as described in Ex. 14, 36 g of crude material are isolated, and respective hydrochloride in ethyl ether (26.3 g) is recrystallized from acetonitrile so give 8.35 g (35 %) of a white solid, m.p. 191-194°C and elemental analysis correct.

IR Spectrum (KBr), $cm^{-1}$: 3100-2400, 1650, 1550, 1440, 1240, 1060, 825.

$^1H$-NMR ($D_2O$), ppm: 0.98 (t, 3H, J=7 Hz; $CH_3$-), 1.32 (d, 3H, J=6.5 Hz; $CH_3$-CH-NH), 1.67 (d + wide band, 5H, J=7 Hz; $CH_3$-and -$CH_2$-), 2.59 (s, 3H; $CH_3$-thiophene), 3.22 (m, 1H; >$\underline{C}H$-NH), 5.06 (m, 1H; >CH-$CH_3$), 7.06 (d, 1H, J=4 Hz; H-thiophene) and 7.96 (d, 1H, J=4 Hz; H-thiophene).

**Example 17**: 2-propionyl-4-methylthiophene

To a solution of 44.92 g of 3-methylthiophene in 250 ml of dry ethyl ether under nitrogen atmosphere and cooled with water bath, 286 ml of n-butyllithium 1.6M solution in hexane are added for about 1 hour. Then, the mixture is refluxed for 2 hours and cooled, and in the course of 30 minutes at -60°C 25.21 g of propionitrile in 115 ml of ethyl ether are added under stirring for 4 hours, and allowed to stand overnight under cooling. Then, the imine is hydrolyzed by slow addition of 230 ml of 2N hydrochloric acid and subsequent distillation of the ethyl ether by heating at 55°C in oil bath. Distillation is stopped and the hydrochloric solution is refluxed for 1 hour. Then, it is cooled, removed twice with 150 ml of ethyl ether and the extracts are washed with water and dried. After evaporation of solvent, the residue weighs 54 g and contains a mixture of 2,4-isomer 75 % and 2,3-isomer 25 %. By distillation, an early richer fraction in 2,3-isomer and 40.4 g are collected at 80-85°C/0.5-0.8 torr (yield: 57 %) containing 81 % of 2-propionyl-4-methylthiophene (GLC).

IR Spectrum (film), $cm^{-1}$: 3100-2800, 1660, 1400, 1230, 1205, 1150, 885, 860, 800.

$^1$H-NMR ($CDCl_3$), ppm: 1.20 (t, 3H, J=7.25 Hz; $CH_3$-), 2.27 (s, 3H, $CH_3$-thiophene), 2.86 (q, 2H, J=7.25 Hz; -$CH_2$-), 7.20 (s, 1H; H-thiophene) and 7.48 (s, 1H; H-thiophene).

**Example 18**: α-Bromo-2-propionyl-4-methylthiophene

From 39.22 g of 2-propionyl-4-methylthiophene (81 %) in 200 ml of methylene chloride and 40.64 g of bromine in the same solvent, and processing as described in Ex. 13, 59.8 g (100 %) of α-bromoderivative are obtained, which is used without further purification.

IR Spectrum (film), $cm^{-1}$: 3100-2900, 1665, 1420, 1240, 1145, 1060, 980, 770.

$^1$H-NMR ($CDCl_3$), ppm: 1.87 (d, 3H, J=7 Hz; $CH_3$-), 2.30 (s, 3H; $CH_3$-thiophene), 5.10 (q, 1H, J=7 Hz; -CHBr-), 7.27 (s, 1H; H-thiophene) and 7.63 (s, 1H; H-thiophene).

**Example 19**: 2-[α-(tert-butylaminopropionyl)]-4-methylthiophene hydrochloride

From 59.61 g of α-bromo-2-propionyl-4-methylthiophene, as described in Ex. 14, in 75 ml of acetonitrile and 46.85 g of tert-butylamine, and processing as described in Ex. 3, 51.2 g of crude material are isolated, and respective hydrochloride in ethyl ether (43.2 g) is recrystallized in ethanol to give 17.8 g (30 %), m.p. 255-258°C (d) and elemental analysis correct.

IR Spectrum (KBr), $cm^{-1}$: 3100-2400, 1665, 1550, 1455, 1250, 1120, 870, 800.

$^1$H-NMR ($D_2O$), ppm: 1.37 (s, 9H; -$C(CH_3)_3$), 1.68 (d, 3H, J=7.0 Hz; $CH_3$-), 1.32 (s, 3H; $CH_3$-thiophene), 5.05 (q, 1H, J=7.0 Hz), 7.76 (s, 1H; H-thiophene) and 8.04 (s, 1H; H-thiophene).

**Example 20**: 2-[α-(iso-propylaminopropionyl)]-4-methylthiophene hydrochloride

From 32.72 g of α-bromo-2-propionyl-4-methylthiophene in 40 ml of acetonitrile and 20.7 g of iso-propylamino, and processing as described m Ex. 14, 26.93 g of crude material are isolated, and respective hydrochloride in ethyl ether (13.5 g) is recrystallized in ethanol to give 7.0 g (25 %), m.p. 230-234°C (d), deprived from 2,3-isomer and elemental analysis correct.

IR Spectrum (KBr), $cm^{-1}$: 3100-2400, 1675, 1445, 1420, 1320, 1230, 1150, 770.

$^1$H-NMR ($D_2O$), ppm: 1.39 (d, 6H, J=6.5 Hz; -$CH(CH_3)_2$), 1.70 (d, 3H, J=7 Hz; $CH_3$-), 2.33 (s, 3H; $CH_3$-thiophene), 3.50 (m, 1H, J=6.5 Hz; -$CH(CH_3)_2$), 5.08 (q, 1H, J=7 Hz; -CO-CH-), 7.71 (s, 1H; H-thiophene) and 7.98 (s, 1H; H-thiophene).

**Example 21**: 4-Bromo-2-methylthiophene

100 g of 4-bromothiophene-2-aldehyde, 193 g of 100 % hydrazine hydrate and 520 ml of ethylene glycol are introduced into a three-necked flask (1-liter capacity) fitted up with a mechanical stirrer and a distillation device. The mixture is heated at 160°C in oil bath which allows to distill the water and the excess of hydrazine; from the distillate the lower phase is separated and introduced into the reactor again.

After completing distillation (6 hours approximately), the mixture is cooled, 117.5 g of potassium hydroxide

(exothermic reaction) are added and then carefully treated till reflux by keeping for 15 minutes. Then, it is allowed to stand overnight end distributed with ethyl ether (250 ml) and water (250 ml); the aqueous phase is removed twice with ether and the organic extracts are washed with 6N hydrochlorid acid and water till neutralization. The crude material is distilled at vacuum and 66.1 g (71.5 %) b.p. 66-73°C/12-5 torr are obtained as colourless liquid.

IR Spectrum (film), $cm^{-1}$: 3120, 3000-2850, 1530, 1330, 1190, 810, 720.

$^1H$ NMR ($CDCl_3$), ppm: 2.44 (s, 3H; $CH_3$-), 6.66 (m, 1H; H-thiophene) and 6.95 (d, 1H, J=1.5 Hz; H-thiophene).

**Example 22**: 2-Methyl-4-propionylthiophene

In a three-nacked flask (1-liter capacity) fitted up with a mechanical stirrer and nitrogen atmosphere, a solution of n-butyllithium is prepered by adding 57.45 g of 1-bromobutane in 50 ml of dry ethyl ether to 4.90 g of lithium in 400 ml of ethyl ether. It is cooled at -70°C (ethanol/$CO_2$ solid) and 38.04 g of 4-bromo-2-methylthiophene in 200 ml of ethyl ether are added along 45 minutes; it is kept for 2 hours at -70°C in order to secure the formation of the lithium salt. Then, at the same temperature, 11.82 g of propionitrile in 50 ml of ethyl ether are added and allowed to stand under stirring for 4 hours and overnight at low temperature. At -15°C, 110 ml of 2N hydrochloric acid are added, the bath is withdrawn and the ether is distilled under pressure; when distillation temperature reaches 100°C, the mixture is refluxed for 1 hour, cooled and the insolubilized oil is removed twice with 200 ml of ethyl ether, then washed with water till neutralization and dried. By distillation of the residue, 12.8 g (40 %) of a colourless liquid are obtained, b.p. 85-100°C/1.5 tors.

IR Spectrum (film), $cm^{-1}$: 3120, 3020-2880, 1680, 1466, 1210, 1145, 860.

$^1H$-NMR ($CDCl_3$), ppm: 1.17 (t, 3H, J=7 Hz; $CH_3$-), 2.46 (s, 3H; $CH_3$-thiophene), 2.82 (q, 2H, J=7 Hz; -$CH_2$-), 7.18 (m, 1H; H-thiophene) and 7.75 (d, 1H, J=1.5 Hz; H-thiophene).

**Example 23**: α-Bromo-2-methyl-4-propionylthiophene

From 12.75 g of 2-methyl-4-propionylthiophene in 65 ml of methylene chloride and 13.22 g of bromine in 35 ml of the same solvent, as described in Ex. 13, 19.4 g (100 %) of α-bromoderivative are obtained and subsequently used without further purification.

IR Spectrum (film), $cm^{-1}$; 3110, 2980, 2930-2860, 1670, 1440, 1220, 1130, 850.

$^1H$-NMR ($CDCl_3$), ppm: 1.81 (d, 3H, J=7 Hz; $CH_3$-), 2.45 (s, 3H; $CH_3$-thiophene), 5.03 (q, 1H, J=7 Hz; CH-), 7.22 (d, 1H, J=1.5 Hz; H-thiophene) and 7.92 (d, 1H, J=1.5 Hz; H-thiophene).

**Example 24**: 4-[α-(tert-butylaminopropionyl)]-2-methylthiophene hydrochloride

From 19.3 g of the α-bromoderivative described in Ex. 23, in 25 ml of acetonitrile and 15.4 g of tert-butylamine, and processing as described in Ex. 14, 13.2 g of crude material are isolated, and respective hydrochloride in ethyl ether (8.0 g) is recrystallized in ethanol to give 4.1 g (22 %), m.p. 241.8-242.4°C and elemental analysis correct.

IR Spectrum (KBr), $cm^{-1}$: 3200-2400, 1670, 1550, 1450, 1230, 1200, 1120, 1010, 840.

$^1H$-NMR ($d_6$-DMSO), ppm: 1.29 (s, 9H; -$C(CH_3)_3$), 1.58 (d, 3H, J=7 Hz; $CH_3$-), 2.48 (d, 3H, J=0.5 Hz; $CH_3$-thiophene), 5.10 (q, 1H, J=7 Hz; >CH-), 7.36 (m, 1H; H-thiophene) and 8.8 (d, 1H, J=1.5 Hz; H-thiophene).

**Example 25**: 4-[α-(iso-propylaminopropionyl)]-2-methylthiophene hydrochloride

To 22.27 g of the α-bromoderivative described in Ex. 23, in 30 ml of acetonitrile and 141.1 g of iso-butylamine, and processing as described in Ex. 14, 16 g of crude material are isolated, and respective hydrochloride is ethyl ether (13 g) is recrystallized in ethanol to give 4.5 g (21 %), in m.p. 231.5-233.5°C and elemental analysis correct.

IR Spectrum (KBr), $cm^{-1}$: 3140-2400, 1665, 1500, 1460, 1210, 1100, 1010, 840, 745.

$^1H$-NMR ($D_2O$), ppm: 1.37 (d, 6H, J=6.5 Hz; -$CH(CH_3)_2$), 1.64 (d, 3H,;=7 Hz; $CH_3$), 2.50 (s, 3H; $CH_3$-thiophene), 3.48 (m, 1H, J=6.5 Hz; -$CH(CH_3)_2$), 5.02 (q, 1H, J=7 Hz; -CO-CH < ), 7.31 (d, 1H, J=1.5 Hz; H-thiophene) and 8.37 (d, 1H, J=1.5 Hz; H-thiophene).

**Example 26**: 2-[α-(iso-propylaminopropionyl)]-4-chlorothiophene hydrochloride

To 63.8 g of α-bromo-4-chloro-2-propionylthiophene obtained according to Example 3 in 70 ml of acetonitrile, 37.2 g of iso-propylamine are gently added without exceeding 32°C. When the exothermia stops, the mixture is left under stirring for 20 hours at room temperature. The solvent is evaporated at vacuum (below 35°C), taken in methylene chloride (150 ml) and water (150 ml); the aqueous phase is removed with methylene chloride, and the organic extracte are washed with water, dried and evaporated to dryness. The crude material (51 g) is dissolved in 400 ml of dry ethyl ether, and by cooling in a water-ice bath a HCl (g) stream is spread; the insolubilized hydrochloride is filtered off, washed with ether and dried at vacuum to give 17.04 g of a white-yellowish solid whose recrystallization in ethanol yields 10.1 g (16 %) of a white solid, m.p. 225-230°C (d), corresponding to the hydrochloride.

IR Spectrum (KBr), $cm^{-1}$: 3140-2400, 1675, 1555, 1400, 1235, 860.

$^1H$-NMR ($d_6$-DMSO), ppm: 1.33 (d, 6H, J=7 Hz; $CH(CH_3)_2$), 1.58 (d, 3H, J=7 Hz; $CH_3$-), 3.38 (m, 1H; $CH(CH_3)_2$), 5.20 (q, 1H, J=7 Hz; CO-CH-), 8.25 (d, 1H, J=1.5 Hz; H-thiophene) and 8.54 (d, 1H, J=1.5 Hz; H-thiophene).

**Example 27**: 2-[α-(n-propylaminopropionyl)]-4-chlorothiophene hydrochloride

Form 36.3 g of α-bromo-4-chloro-2-propionylthiophene in 45 ml of acetonitrile and 20.42 g of n- propylamine, and processing as described in the above examples, 32.05 g of crude material are isolated, and respective hydrochloride (31.5 g) in ethyl ether is recrystallized in ethyl acetate/acetonitrile to give 12.7 g (33 %) of a white solid, m.p. 192.5-193.5°C and elemental analysis correct.

IR Spectrum (KBr), $cm^{-1}$: 3100-2400, 1680, 1440, 1405, 1230, 1190, 1110, 940, 770.

$^1H$-NMR ($CD_3OD$), ppm: 0.99 (t, 3H, J=7 Hz; $CH_3$-), 1.62 (d, 3H, J=7 Hz; $CH_3$-), 1.76 (m, 2H, J=7 Hz; $CH_2$-$CH_3$), 3.0 (m, 2H; $CH_2$-NH), 5.00 (q, 1H, J=7 Hz; CH-CO), 7.90 (d, 1H, J=1.5 Hz; H-tiophene) and 8.05 (d, 1H, J=1.5 Hz; H-thiophene).

## Claims

1. Thiophene ring-substituted α-(alkylaminopropionyl)-thiophene derivatives having the general formula (I):

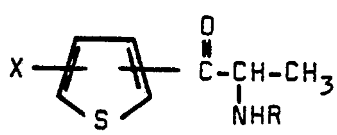

(I)

wherein X is methyl or chlorine,

R is alkyl, linear or branched, having 3 to 4 carbon atoms, and the nontoxic addition salts thereof.

2. A process for preparing the compounds according to Claim 1, characterized in that an α-(alkylaminopropionyl)-thiophene of the general formula (II):

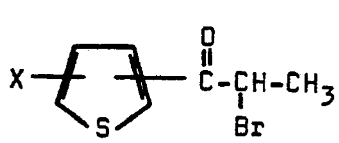

(II)

wherein X has the same meaning as defined in claim 1, is reacted with an amine of the general formula (III):

$H_2N$-R (III)

wherein R has the same meaning as defined in claim 1, in an inert medium, and at a maximal temperature of 50°C, and, if desired, the free base is treated with an acid to obtain a non-toxic addition salt, or a salt of a compound of the general formula I is neutralised to obtain the free base.

3. The process of Claim 2, characterized in that acetonitrile is used as inert medium in the reaction between

(II) and (III).

4. The process of Claim 2, characterized in that a chlorinated hydrocarbon or an ether is used as inert medium in the formation of the nontoxic addition salts.

5. The process of Claim 4, wherein the chlorinated hydrocarbon is methylene chloride and the ether is diethyl ether.

6. Pharmaceutical composition comprising at least one of the compounds of Claim 1, optionally together with pharmaceutical carriers and/or adjuvants.

7. The use of the compounds according to claim 1 for the preparation of pharmaceutical compositions for treating depressions.

**Revendications**

1. Dérivés d'α-(alkylaminopropionyl)thiophène substitués dans le noyau thiophène, répondant à la formule generale (I):

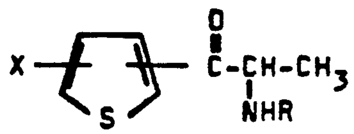

(I)

dans laquelle X est un groupe méthyle ou un atome de chlore, R est un groupe alkyle, linéaire ou ramifié, en $C_3$ à $C_4$, et leurs sels d'addition non toxiques.

2. Procédé pour préparer les composés selon la revendication 1, caractérisé en ce qu'on fait réagir un α-(alkylaminopropionyl)-thiophène répondant à la formule générale (II):

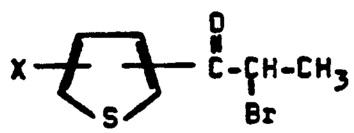

(II)

dans laquelle X a la même signification que dans la revendication 1, avec une amine répondant à la formule générale (III)

$H_2N$-R (III)

dans laquelle R a la même signification que dans la revendication 1, dans un milieu inerte, et à une température maxima de 50°C, et, si on le désire, on traite la base libre par un acide pour obtenir un sel d'addition non toxique, ou on neutralise un sel d'un composé répondant à la formule générale I pour obtenir la base libre.

3. Procédé selon la revendication 2, caractérisé en ce qu'on utilise de l'acétonitrile comme milieu inerte, dans la réaction entre (II) et (III).

4. Procédé selon la revendication 2, caractérisé en ce qu'on utilise un hydrocarbure chloré ou un éther comme milieu inerte dans la formation des sels d'addition non toxiques.

5. Procédé selon la revendication 4 dans lequel l'hydrocarbure chloré est le chlorure de méthylène et l'éther est l'éther diéthylique.

6. Composition pharmaceutique comprenant au moins un des composés selon la revendication 1, en même temps si on le désire que des supports et/ou adjuvants pharmaceutiques.

7. Utilisation des composés selon la revendication 1 pour la préparation de compositions pharmaceutiques pour le traitement des dépressions.

**Patentansprüche**

1. Thiophenring-substituierte α-(Alkylaminopropionyl)-thiophenderivate der allgemeinen Formel (I):

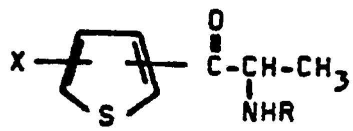

(I)

worin
X für Methyl oder ein Chloratom steht,
R für einen linearen oder verzweigten Alkylrest mit 3 bis 4 Kohlenstoffatomen steht,
und deren nicht-toxische Additionssalze.

2. Verfahren zur Herstellung der Verbindungen gemäß Anspruch 1, dadurch gekennzeichnet, daß ein α-(Alkylaminopropionyl)-thiophen der allgemeinen Formel (II):

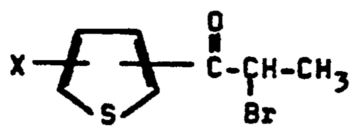

(II)

worin X die in Anspruch 1 angegebenen Bedeutungen besitzt, mit einem Amin der allgemeinen Formel (III):

$H_2N$-R (III)

worin R die in Anspruch 1 angegebenen Bedeutungen besitzt, in einem inerten Medium und bei einer maximalen Temperatur von 50°C umgesetzt wird, und gegebenenfalls die freie Base mit einer Säure zur Bildung eines nicht-toxischen Additionssalzes umgesetzt wird, oder ein Salz einer Verbindung der allgemeinen Formel (I) zur Bildung der freien Base neutralisiert wird.

3. Verfahren nach Anspruch 2, dadurch gekennzeichnet, daß Acetonitril als inertes Medium bei der Reaktion von (II) und (III) verwendet wird.

4. Verfahren nach Anspruch 2, dadurch gekennzeichnet, daß ein chlorierter Kohlenwasserstoff oder ein Äther als inertes Medium bei der Bildung des nicht-toxischen Additionssalzes verwendet wird.

5. Verfahren nach Anspruch 4, wobei als chlorierter Kohlenwasserstoff Methylenchlorid und als Äther Diethyläther verwendet wird.

6. Pharmazeutisches Mittel, welches mindestens eine der Verbindungen aus Anspruch 1, gegebenenfalls zusammen mit pharmazeutischen Trägern und/oder Adjuvanzien umfaßt.

7. Verwendung der Verbindungen aus Anspruch 1 zur Herstellung von pharmazeutischen Mitteln zur Behandlung von Depressionen.